(19) Europäisches Patentamt / European Patent Office / Office européen des brevets

(11) **EP 3 960 085 B1**

(12) **EUROPEAN PATENT SPECIFICATION**

(45) Date of publication and mention
of the grant of the patent:
**02.08.2023 Bulletin 2023/31**

(21) Application number: **21194349.3**

(22) Date of filing: **01.09.2021**

(51) International Patent Classification (IPC):
*A61B 5/367* (2021.01)  *A61B 5/287* (2021.01)
*A61B 5/349* (2021.01)  *A61B 5/06* (2006.01)
*A61B 5/00* (2006.01)  *G06N 3/045* (2023.01)
*G06N 3/084* (2023.01)  *G16H 20/40* (2018.01)
*G16H 50/20* (2018.01)  *G16H 50/70* (2018.01)
*A61B 5/361* (2021.01)  *A61B 5/363* (2021.01)
*G06N 3/044* (2023.01)  *G06N 3/048* (2023.01)

(52) Cooperative Patent Classification (CPC):
**A61B 5/367; A61B 5/061; A61B 5/287;**
**A61B 5/349; A61B 5/7267; G06N 3/045;**
**G06N 3/084; G16H 20/40; G16H 50/20;**
**G16H 50/70;** A61B 5/0006; A61B 5/063;
A61B 5/361; A61B 5/363; G06N 3/044;  (Cont.)

(54) **ARRHYTHMIA CLASSIFICATION FOR CARDIAC MAPPING**

ARRHYTHMIEKLASSIFIZIERUNG FÜR DIE KARDIALE KARTIERUNG

CLASSIFICATION D'ARYTHMIE POUR LA CARTOGRAPHIE CARDIAQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.09.2020  US 202063073217 P**
**12.10.2020  US 202063090494 P**
**30.08.2021  US 202117460863**

(43) Date of publication of application:
**02.03.2022 Bulletin 2022/09**

(73) Proprietor: **Biosense Webster (Israel) Ltd**
**Yokneam, 2066717 (IL)**

(72) Inventors:
• **Goldberg, Stanislav**
**Yokneam (IL)**
• **Amit, Matityahu**
**Yokneam (IL)**
• **Auerbach, Smhuel**
**Yokneam (IL)**
• **Amos, Yariv Avraham**
**Yokneam (IL)**
• **Yarnitsky, Jonathan**
**Yokneam (IL)**
• **Urman, Roy**
**Yokneam (IL)**

(74) Representative: **Carpmaels & Ransford LLP**
**One Southampton Row**
**London WC1B 5HA (GB)**

(56) References cited:
**US-A1- 2013 096 449    US-A1- 2015 065 836**
**US-A1- 2019 223 808**

(52) Cooperative Patent Classification (CPC): (Cont.)
G06N 3/048

**Description**

BACKGROUND

An electrophysiology (EP) procedure is an assessment of an electrical activity in the heart that is used to diagnose an abnormal heartbeat or arrhythmia. EP procedures may be performed by utilizing body surface electrodes or intracardiac electrodes (where a catheter is inserted through blood vessels into the heart to measure electrical activity). EP procedures provide cardiac images that are generated based on the measured electrical activity, including images of the cardiac tissue, chambers, veins, arteries and/or pathways. Conventional cardiac images can visualize electrical activity in the heart by measurements of electrical potentials, presenting the voltage of beats (propagating pulses generated by the arrhythmia) at various tissue locations, that is, a voltage map. Cardiac images can also visualize electrical activity by measurements of local activation times (LAT), presenting the arrival time of the beats to various tissue locations, that is, a LAT map.

An inherent assumption of cardiac imaging is that the type of arrhythmia that generates the electrical activity captured by the cardiac imaging does not change rapidly. Yet, in practice, during an EP procedure, a patient may endure arrhythmia that changes its nature rapidly and spontaneously. Therefore, heart mapping (either voltage mapping or LAT mapping) may involve visualization of data from beats that were originated by different arrhythmia types. Such erroneous heart mapping affects the clinical interpretation of the arrhythmia condition. A reliable technique is needed to ensure that during an EP procedure cardiac images each visualizes electrical activities that were generated by arrythmia of one class.

US 2015/0065836 A1 discloses a system and method for mapping an anatomical structure, which includes sensing activation signals of physiological activity with a plurality of mapping electrodes disposed in or near the anatomical structure. Patterns among the sensed activation signals are identified based on a similarity measure generated between each unique pair of identified patterns which are classified into groups based on a correlation between the corresponding pairs of similarity measures. A characteristic representation is determined for each group of similarity measures and displayed as a summary plot of the characteristic representations.

US 2013/0096449 A1 discloses the classification of cardiac episodes using an algorithm. In various examples, an episode classification algorithm evaluates electrogram signal data using a probabilistic ventricular oversensing algorithm. The algorithm may look at a plurality of factors weighing for and against a determination of ventricular oversensing. In some examples, the algorithm may also determine whether the cardiac episode includes atrial sensing issues.

US 2019/0223808 A1 discloses the classification of heartbeats based on intracardiac and body surface electrocardiogram (ECG) signals. Intracardiac ECG (IC-ECG) signals and body surface ECG (BS-ECG) signals are processed to perform heartbeat classifications. A BS annotation of BS-ECG signals reflective of a sensed heartbeat is defined, the BS annotation including a BS annotation time value. IC annotations of IC-ECG signals which reflect atrial-activity or ventricular activity of the sensed heartbeat are also defined, each IC annotation including an IC annotation time value. The IC-ECG signals are discriminated as A-activity or V-activity and IC annotations are designated as IC-A annotations or IC-V annotations, respectively. A respective A/V time sequence comparison of IC annotations reflective of the sensed heartbeat is made with one or more time sequence templates for heartbeat classification. Morphology comparisons of the BS-ECG oscillating signal segments reflective of the sensed heartbeat morphology templates for classification may also be made.

SUMMARY

The present invention provides systems and a non-transitory computer readable medium for cardiac mapping as claimed hereinafter.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 is a diagram of an example cardiac system, based on which one or more features of the disclosure may be implemented;
FIG. 2 is a block diagram of an example system, deployable by the example cardiac system of FIG. 1, based on which one or more features of the disclosure may be implemented;
FIG. 3 is a functional block diagram of an example system for cardiac mapping of data according to clusters, based on which one or more features of the disclosure may be implemented;
FIG. 4 is a flow chart of an example method for cardiac mapping of data according to clusters, based on which one or more features of the disclosure may be implemented;

FIG. 5 is a flow chart of an example method for classifying beat segments into clusters based on beat characteristics, based on which one or more features of the disclosure may be implemented;

FIG. 6 is a diagram of an example clustering method, based on which one or more features of the disclosure may be implemented;

FIG. 7 is a flow chart of an example method for classifying beat segments into clusters based on a prediction of an arrhythmia type, including a training method of a predictor (FIG. 7A) and application method of the trained predictor (FIG. 7B), based on which one or more features of the disclosure may be implemented; and

FIG. 8 is a functional block diagram of an example machine learning system, based on which one or more features of the disclosure may be implemented.

DETAILED DESCRIPTION

Disclosed herein are systems for cardiac mapping of data according to clusters. Clusters can be formed based on arrhythmia type or electrocardiogram (ECG) data characteristics, for example. Data associated with a cluster can then be used to create a cardiac map that visualizes the electrical activity corresponding to the arrythmia type or to the ECG data characteristics associated with the cluster. In aspects herein, biometric data, measured from a patient during an EP procedure (e.g., including body surface (BS) ECG data, intracardiac electrocardiogram (IC) ECG data, ablation data, and catheter electrode position data) are processed. Beat segments extracted from ECG data are classified into clusters based on the arrhythmia type that produced them. The beat segments can be classified into clusters based on a predicted arrhythmia type or based on beat characteristics. Thus, cardiac maps, corresponding to arrythmia clusters, can be generated, that is, biometric data associated with a cluster can be visualized in each cardiac map.

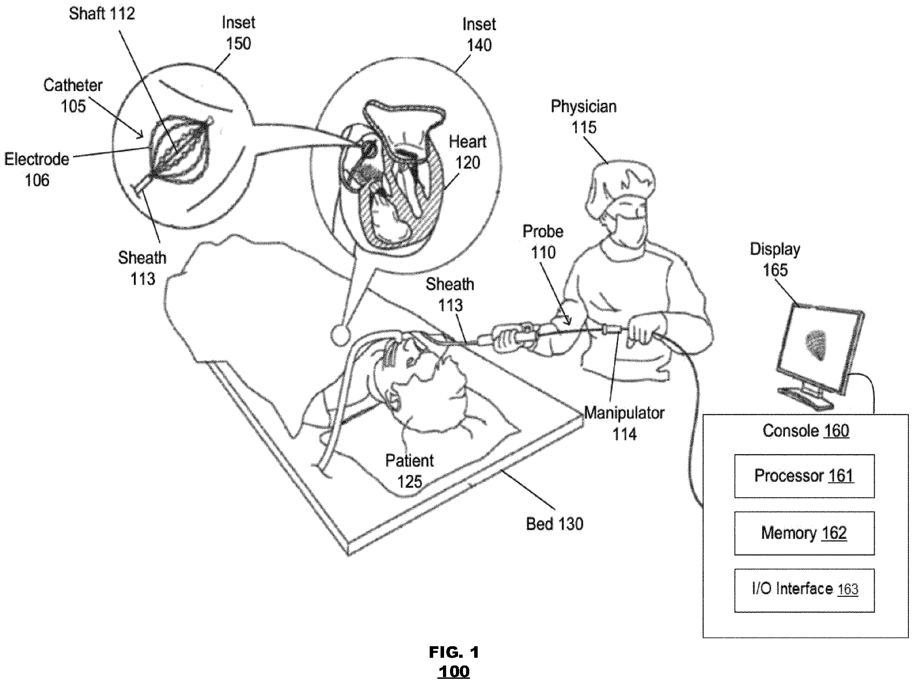

FIG. 1 is a diagram of an example cardiac system 100, based on which one or more features of the disclosure may be implemented. The system 100 may be used to collect, process, and visualize biometric data associated with the heart 120 of a patient 125 shown laying on a surface 130 during a cardiac procedure performed by a physician (or a medical professional) 115. The system 100 may include a probe 110 connected to a console 160 and a display 165. The probe 110 may include a catheter 105 (having at least one electrode 106), a shaft 112, a sheath 113, and a manipulator 114. Inset 150 shows the catheter 105 in an enlarged view and inset 140 shows the catheter 105 inside a cardiac chamber of the heart 120. Note that each element and/or item of the system 100 is representative of one or more of that element and/or that item. The example of the system 100 shown in FIG. 1 may be modified to implement aspects disclosed herein. Aspects disclosed herein may similarly be applied using other system components and settings.

The system 100 may be utilized to detect, diagnose, and treat cardiac conditions, according to aspects disclosed herein. Cardiac conditions, such as cardiac arrhythmias persist as common and dangerous medical conditions, especially in the aging population. In patients with normal sinus rhythm, the heart (which includes atrial, ventricular, and excitatory conduction tissue) is electrically excited to beat in a synchronous, patterned fashion. This electrical excitation may be detected as intracardiac signals. In patients with a cardiac arrhythmia, abnormal regions of cardiac tissue do not follow the synchronous beating cycle associated with normally conductive tissue as in patients with a normal sinus rhythm. In those patients with a cardiac arrhythmia, the abnormal regions of cardiac tissue aberrantly conduct to adjacent tissue, thereby disrupting the cardiac cycle into an asynchronous cardiac rhythm. This asynchronous cardiac rhythm may also be detected through intracardiac signals. Such abnormal conductions have been previously known to occur at various regions of the heart, for example, in the region of the sino-atrial (SA) node, along the conduction pathways of the atrioventricular (AV) node, or in the cardiac muscle tissue forming the walls of the ventricular and atrial cardiac chambers.

The catheter 105 may be configured to obtain biometric data including electrical signals of an intra-body organ (e.g., the heart 120) and/or to ablate tissue areas of a cardiac chamber of the heart 120. A catheter's electrode 106 may represent any element, such as tracking coils, piezoelectric transducer, electrodes, or combination of elements that may be configured to ablate the tissue areas or to obtain the biometric data associated with the heart. For example, the catheter 105 may use its electrodes 106 to generate intravascular ultrasound and/or MRI based images. In another example, the catheter 105 may be used to ablate cardiac tissues by targeting those tissues with energy. The energy may be thermal energy and may cause damage to the tissue area starting from the surface of the tissue area and extending into the thickness of these tissue area.

The console 160 of the system 100 may include a processor 161, memory 162, and an I/O interface 163. The processor 161 may represent one or more processors 161 configured to execute software modules associated with the training and the application of an arrhythmia predictor, according to aspects described herein. The memory 162 may represent any non-transitory tangible media that may facilitate storage of computer instructions and data associated with the execution of the software modules. In an aspect, the execution of one or more of the software modules associated with the training and the application of an arrhythmia predictor may be external to the console 160, for example, in the catheter 105 or other external devices (e.g., a mobile device, a cloud-based device, or a standalone processor). In such a case, one or more of the software modules may be transferable over a network. The I/O interface 163 may enable transmitting and receiving data to and from the catheter 105 as well as transmitting and receiving control messages among components of the system 100. For example, the I/O interface 163 may enable the console 160 to receive signals from and/or transfer signals to at least one electrode 106 of the catheter 105. The console 160 may include real-time noise reduction circuitry, typically configured as a field programmable gate array (FPGA), followed by an analog-to-digital (A/D) electrocardiograph or electromyogram (EMG) signal conversion integrated circuit. The console 160 may process and/or store the signal from the A/D ECG or EMG circuit or may transmit it to an external device for processing and/or storage.

The display 165 of the system 100 may be connected to the console 160. During a procedure, the console 160 may facilitate the visualization (rendering) of a body part on the display 165 to be viewed by the physician 115, and may store rendered body part data in the memory 162. In an aspect, the physician 115 may be able to manipulate the rendered body part using one or more input devices, such as a touch pad, a mouse, a keyboard, or a gesture recognition apparatus. For example, an input device may be used to change a position of the catheter 105 such that the rendering of the body part is updated. The display 165 may include a touchscreen that can be configured to accept other inputs from the medical professional 115, in addition to inputs for controlling the rendering of the body part. Note that the display 165 may be positioned local to the console 160 or at a remote location, such as another hospital or healthcare provider site. Additionally, the system 100 may be part of a surgical system that is configured to obtain anatomical and electrical measurements of a patient's organ, such as the heart, and performing a cardiac ablation procedure. An example of such a surgical system is the Carto® system sold by Biosense Webster.

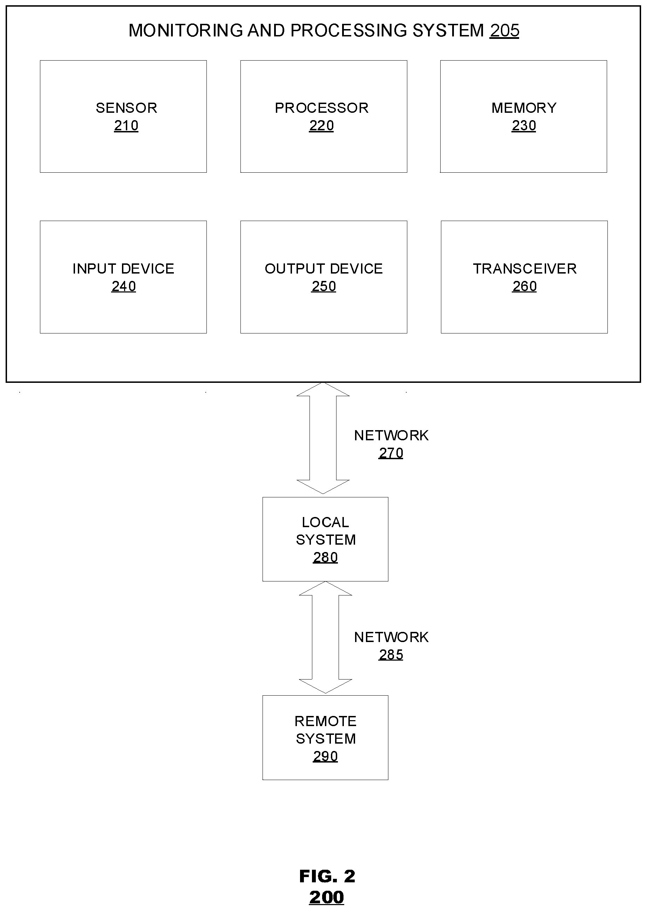

FIG. 2 is a block diagram of an example system 200, deployable by the example cardiac ablation system of FIG. 1, based on which one or more features of the disclosure may be implemented. The system 200 may include a monitoring and processing system 205, a local system 280, and a remote system 290. In an alternative, the monitoring and processing system 205 may represent the console 160 of system 100. The monitoring and processing system 205 may include a patient biometric sensor 210, a processor 220, memory 230, an input device 240, an output device 250, and a transceiver 260, i.e., a transmitter-receiver that is in communication with a network 270. The system 205 may continually or periodically monitor, store, process, and communicate, via the network 270, various patient biometrics. Examples of patient biometrics include electrical signals (e.g., ECG signals), anatomical images, blood pressure data, blood glucose data, and temperature data. The patient biometrics may be monitored and may be communicated for treatment of various diseases, such as cardiovascular diseases (e.g., arrhythmias, cardiomyopathy, and coronary artery disease) and autoimmune diseases (e.g., type I and type II diabetes).

The monitoring and processing system 205 may be internal to the patient's body - e.g., the system 205 may be subcutaneously implantable, inserted orally or surgically, via a vein or an artery, via an endoscopic or a laparoscopic procedure. Alternatively, the system 205 may be external to the patient, e.g., attached to the patient's skin. In an aspect, the system 205 may include both components that are internal to the patient's body and components that are external to the patient's body.

The monitoring and processing system 205, may represent a plurality of monitoring and processing systems 205 that may process biometric data of a patient in parallel and/or in communication with each other or in communication with a server via a network. One or more systems 205 may acquire or receive all or part of a patient's biometric data (e.g., electrical signals, anatomical images, blood pressure, temperature, blood glucose level, or other biometric data). The one or more systems 205 may also acquire or receive additional information associated with the acquired or received patient's biometric data from one or more other systems 205. The additional information may be, for example, diagnosis information and/or information obtained from a device such as a wearable device. Each monitoring and processing system 205 may process data acquired by itself and may process data received from another system 205.

The patient biometric sensor 210 may be one or more sensors that may be configured to sense biometric data. For example, the sensor 210 may be an electrode configured to acquire electrical signals (e.g., bioelectrical signals originating in the heart), a temperature sensor, a blood pressure sensor, a blood glucose sensor, a blood oxygen sensor, a pH sensor, an accelerometer, or a microphone. In an aspect, system 205 may be an ECG monitor that measures ECG signals originating in the heart. In such a case, the sensor 210 may be one or more electrodes that may be configured to acquire the ECG signals. The ECG signals may be used for treatment of various cardiovascular diseases. In an aspect, the patient biometric sensor 210 may also include a catheter with one or more electrodes, a probe, a blood pressure cuff, a weight scale, a bracelet (e.g., a smart watch biometric tracker), a glucose monitor, a continuous positive airway pressure (CPAP) machine, or any other device that provides biometric data or other data concerning the patient health.

The transceiver 260 may include a transmitter component and a receiver component. These transmitter and receiver components may be integrated into a single device or separately implemented. The transceiver may provide connectivity between the system 205 and other systems or servers via a communication network 270. The network 270 may be a wired network, a wireless network or include a combination of wired and/or wireless networks. The network 270 may be a short-range network (e.g., a local area network (LAN) or a personal area network (PAN)). Information may be sent or may be received via the short-range network using various short-range communication protocols such as Bluetooth, Wi-Fi, Zigbee, Z-Wave, near field communications (NFC), ultra-band, Zigbee, or infrared (IR). The network 270 may also be a long-range network (e.g., wide area network (WAN), the internet, or a cellular network). Information may be sent or may be received via the long-range network using various long-range communication protocols such as TCP/IP, HTTP, 3G, 4G/LTE, or 5G/New Radio.

The processor 220 may be configured to process patient's biometric data, obtained by the sensor 210 for example, and store the biometric data and/or the processed biometric data in memory 230. The processor 220 may also be configured to communicate the biometric data across the network 270 via a transmitter of the transceiver 260. Biometric data from one or more other monitoring and processing systems 205 may be received by a receiver of transceiver 260. The processor 220 may employ a machine learning algorithm (e.g., based on a neural network), or, alternatively, a machine learning algorithm may be employed by another processor, e.g., at the local system 280 or the remote system 290. In aspects, the processor 220 may include one or multiple CPUs, one or multiple GPUs, or one or multiple FPGAs. In these aspects, the machine learning algorithm may be executed on one or more of these processing units. Similarly, the processor 220 may include an ASIC dedicated to performing deep learning calculations (such as the Intel® Nervana™ Neural Network Processor) and the machine learning algorithm may be executed on such dedicated ASIC. The processing unit that executes the machine learning algorithm may be located in the medical procedure room or in another location (e.g., another medical facility or a cloud).

The input device 240 of the monitoring and processing system 205 may be used as a user interface. The input device 240 may include, for example, a piezoelectric sensor or a capacitive sensor that is configured to receive user input, such as tapping or touching. Hence, the input device 240 may be configured to implement capacitive coupling in response to tapping or touching a surface of the system 205 by a user. Gesture recognition may be implemented by various capacitive coupling such as resistive capacitive, surface capacitive, projected capacitive, surface acoustic wave, piezoelectric, or infra-red touching. Capacitive sensors may be placed on the surface of the input device 240 so that the tapping or touching of the surface activates the system 205. The processor 220 may be configured to respond selectively to different tapping patterns of the capacitive sensor (e.g., a single tap or a double tap on the input device 240) such that different functions of the system 205 (e.g., acquisition, storing, or transmission of data) may be activated based on the detected pattern. In an aspect, audible feedback may be given to the user from the system 205, e.g., when a gesture is detected and recognized.

In an aspect, the local system 280, that may be in communication with the monitoring and processing system 205 via the network 270, may be configured to act as a gateway to a remote system 290 through another network 285 that may be accessible to the local system 280. The local system 280 may be, for example, a smart phone, smartwatch, tablet, or other portable smart device. Alternatively, the local system 280 may be a stationary or a standalone device. Patient biometric data may be communicated between the local system 280 and the monitoring and processing system 205. In an aspect, the local system 280 may also be configured to display the acquired patient biometric data and associated information.

In an aspect, the remote system 290 may be configured to receive at least part of the monitored patient biometric data and associated information via the network 285, which may be a long-range network. For example, if the local system 280 is a mobile phone, network 285 may be a wireless cellular network. Information may be communicated between the local system 280 and the remote system 290 via a wireless technology standard, such as any of the wireless technologies mentioned above. The remote system 290 may be configured to present received patient biometric data and associated information to a healthcare professional (e.g., a physician), either visually on a display or aurally through a speaker.

As described in reference to FIG. 1 and FIG. 2, the system 100, 200 can process 161, 220 biometric data associated with a patient. The system 100, 200 can receive 163, 260 the biometric data from measurements performed on a patient 125 during an EP procedure. The system 100 may also, and optionally, obtain biometric data such as anatomical measurements of the heart 120 using ultrasound, computed tomography (CT), MRI, or other medical imaging techniques known in the art. The system 100 may obtain ECGs or electrical measurements using catheters or other sensors that measure electrical properties of the heart 120. The biometric data including anatomical and electrical measurements may then be stored in the memory 162 of the console 160. The biometric data may be transmitted to the console 160 from the non-transitory tangible media. Alternatively, or in addition, the biometric data may be transmitted to a server, which may be local or remote, using a network as further described herein.

According to an aspect, the console 160 may be connected, by a cable, to body surface electrodes, which may include adhesive skin patches that are affixed to the patient 125. The body surface electrodes can procure/generate the biometric data in the form of BS ECG data. For instance, the processor 161, in conjunction with a current tracking module, may determine position coordinates of the catheter 105 inside the body part (e.g., the heart 120) of the patient 125. The position coordinates may be based on impedances or electromagnetic fields measured between the body surface electrodes and the electrode 106 of the catheter 105 or other electromagnetic components. Additionally, or alternatively, location pads may be located on a surface of the bed 130 and may be separate from the bed 130.

According to aspects, the catheter 105 may be configured to ablate tissue areas of a cardiac chamber of the heart 120. Inset 150 shows the catheter 105 in an enlarged view, inside a cardiac chamber of the heart 120. According to aspects disclosed herein, the ablation electrodes, such as the at least one electrode 106, may be configured to provide energy to tissue areas of an intra-body organ such as heart 120. The energy may be thermal energy and may cause damage to the tissue area starting from the surface of the tissue area and extending into the thickness of the tissue area. Biometric data with respect to ablation procedures (e.g., ablation tissues, ablation locations, etc.) can be considered ablation data.

According to one or more aspects, catheters containing position sensors may be used to determine the trajectory of points on the cardiac surface. These trajectories may be used to infer motion characteristics such as the contractility of the tissue. Maps depicting such motion characteristics may be constructed when the trajectory information is sampled at a sufficient number of points in the heart 120.

Electrical activity at a point in the heart 120 may be typically measured by advancing the catheter 105 containing an electrical sensor at or near its distal tip (e.g., the at least one electrode 106) to that point in the heart 120, contacting the tissue with the sensor and acquiring data at that point. One drawback with mapping a cardiac chamber using the catheter 105 containing only a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

According to an example, a multi-electrode catheter may be advanced into a chamber of the heart 120. Anteroposterior (AP) and lateral fluorograms may be obtained to establish the position and orientation of each of the electrodes. ECGs may be recorded from each of the electrodes in contact with a cardiac surface relative to a temporal reference such as the onset of the P-wave in sinus rhythm from a BS ECG. The system, as further disclosed herein, may differentiate between those electrodes that register electrical activity and those that do not due to absence of close proximity to the endocardial wall. After initial ECGs are recorded, the catheter may be repositioned, and fluorograms and ECGs may be recorded again. An electrical map may then be constructed from iterations of the process above.

According to an example, cardiac mapping may be generated based on detection of intracardiac electrical potential fields (e.g., which is an example of IC ECG data). A non-contact technique to simultaneously acquire a large amount of cardiac electrical information may be implemented. For example, a catheter having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes).

According to another example, an electrophysiological cardiac mapping system and technique based on a non-contact and non-expanded multi-electrode catheter may be implemented. ECGs may be obtained with catheters having multiple electrodes (e.g., between 42 to 122 electrodes). According to this implementation, knowledge of the relative geometry of the probe and the endocardium may be obtained such as by an independent imaging modality such as transesophageal echocardiography. After the independent imaging, non-contact electrodes may be used to measure cardiac surface potentials and construct maps therefrom. This technique may include the following steps (after the independent imaging step): (a) measuring electrical potentials with a plurality of electrodes disposed on a probe positioned in the heart 120; (b) determining the geometric relationship of the probe surface and the endocardial surface; (c) generating a matrix of coefficients representing the geometric relationship of the probe surface and the endocardial surface; and (d) determining endocardial potentials based on the electrode potentials and the matrix of coefficients.

According to another example, a technique and apparatus for mapping the electrical potential distribution of a heart chamber may be implemented. An intra-cardiac multi-electrode mapping catheter assembly may be inserted into the heart 120. The mapping catheter assembly may include a multi-electrode array with an integral reference electrode, or, preferably, a companion reference catheter. The electrodes may be deployed in the form of a substantially spherical array. The electrode array may be spatially referenced to a point on the endocardial surface by the reference electrode or by the reference catheter which is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

According to another example, a heart mapping catheter assembly may include an electrode array defining a number of electrode sites. The mapping catheter assembly may also include a lumen to accept a reference catheter having a distal tip electrode assembly which may be used to probe the heart wall. The mapping catheter may include a braid of insulated wires (e.g., having 24 to 64 wires in the braid), and each of the wires may be used to form electrode sites. The catheter may be positioned at a first site in the heart 120 to acquire a first set of electrical activity information from non-contact electrodes and then may be positioned at another site in the heart 120 to acquire another set of electrical activity information from the non-contact electrodes.

According to another example, another catheter for mapping electrophysiological activity within the heart may be implemented. The catheter body may include a distal tip which is adapted for delivery of a stimulating pulse for pacing the heart or an ablative electrode for ablating tissue in contact with the tip. The catheter may further include at least one pair of orthogonal electrodes to generate a difference signal indicative of the local cardiac electrical activity adjacent the orthogonal electrodes.

According to another example, a process for measuring electrophysiologic data in a heart chamber may be implemented. The method may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred aspects, the array is said to have from 60 to 64 electrodes.

According to another example, cardiac mapping may be implemented using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of a probe (e.g., a treatment catheter) at the later time may be displayed and the probe may be overlaid onto the one or more ultrasound slices.

According to other examples, body patches and/or body surface electrodes may be positioned on or proximate to a patient's body. A catheter with one or more electrodes may be positioned within the patient's body (e.g., within the patient's heart 120) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient 125 (e.g., within the heart 120). The biometric data may be associated with the determined position of the catheter such that a rendering of the patient's body part (e.g., the heart 120) may be displayed and may show the biometric data overlaid on a shape of the body.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of the IC ECG data). Alternatively, or in addition to the multiwavelet reentrant type, cardiac arrhythmias may also have a focal origin, such as when an isolated region of tissue in an atrium fires autonomously in a rapid, repetitive fashion (e.g., another example of the IC ECG data). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

For example, aFib occurs when the normal electrical impulses (e.g., another example of the IC ECG data) generated by the sinoatrial node are overwhelmed by disorganized electrical impulses (e.g., signal interference) that originate in the atria veins and PVs causing irregular impulses to be conducted to the ventricles. An irregular heartbeat results and may last from minutes to weeks, or even years. aFib is often a chronic condition that leads to a small increase in the risk of death often due to strokes. The first line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Cardiac mapping (which is an example of heart imaging) includes creating a map of electrical potentials (e.g., a voltage map) of the wave propagation along the heart tissue or a map of arrival times (e.g., a LAT map) to various tissue located points. Cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by the formation of non-conducting lesions. Various energy delivery modalities have been disclosed for forming lesions, and include use of microwave, laser and more commonly, radiofrequency energies to create conduction blocks along the cardiac tissue wall. In a two-step procedure (e.g., mapping followed by ablation) electrical activity at points within the heart 120 is typically sensed and measured by advancing the catheter 105 containing one or more electrical sensors (e.g., electrodes 106) into the heart 120 and obtaining/acquiring data at a multiplicity of points (e.g., ECG data). This ECG data is then utilized to select the endocardial target areas, at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the classification engine 101 employed by the system 100 herein manipulates and evaluates the ECG data to produce improved tissue data that enables more accurate diagnosis, images, scans, and/or maps for treating an abnormal heartbeat or arrhythmia.

Electrode catheters (e.g., the catheter 105) are used in medical practice. Electrode catheters are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. Radio frequency (RF) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees Celsius, a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter must be removed from the body and the tip electrode cleaned.

Treatments for cardiac conditions such as cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, modules that are executed by the system 100, 200 may be executed by the catheter 105.

Mapping of cardiac areas such as cardiac regions, tissue, veins, arteries and/or electrical pathways of the heart (e.g., 120) may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to, local activation time (LAT), an electrical activity, a topology, a bipolar mapping, a dominant frequency, or an impedance. Data corresponding to multiple modalities may be captured using a catheter inserted into a patient's body and may be provided for rendering at the same time or at different times based on corresponding settings and/or preferences of a medical professional.

Cardiac mapping may be implemented using one or more techniques. As an example of a first technique, cardiac mapping may be implemented by sensing an electrical property of heart tissue, for example, LAT, as a function of the precise location within the heart. The corresponding data may be acquired with one or more catheters that are advanced into the heart using catheters that have electrical and location sensors in their distal tips. As specific examples, location and electrical activity may be initially measured on about 10 to about 20 points on the interior surface of the heart. These data points may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

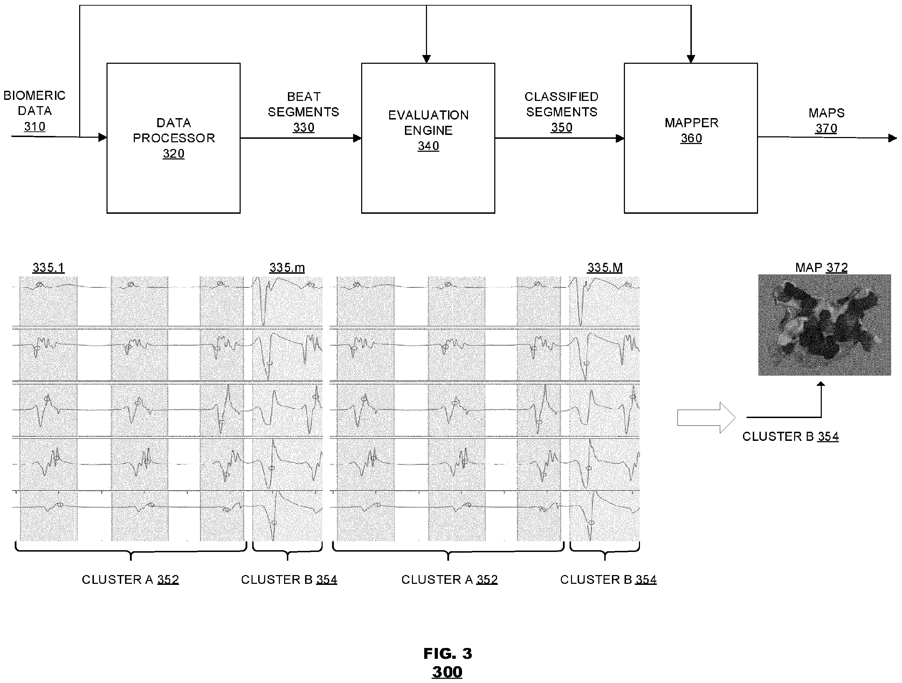

FIG. 3 is a functional block diagram of an example system for cardiac mapping of data according to clusters 300, based on which one or more features of the disclosure may be implemented. According to aspects disclosed herein, the system 300 may classify ECG data of biometric data measured from a patient into clusters and map the biometric data associated with each cluster. The system 300 may include a data processor 320, an evaluation engine 340, and a mapper 360. The system's 300 components 320, 340, 360, may be employed by one or more software modules executable by one or more processors of the system 100, 200, described above with reference to FIG.1 and FIG.2. The data processor 320 may receive as an input biometric data 310 containing data collected from a patient during a medical procedure. The data processor 320 may process the received biometric data 310, for example, to improve the discriminatory properties of these data and/or to reduce the data size. The data processor 320 may feed the evaluation engine 340 with beat segments 335.1-335.M extracted from ECG data of the biometric data 310. Each beat segment (e.g., including ECG signals 335.m) is representative of the arrhythmia type it was produced from.

The evaluation engine 340 may classify the beat segments 330 into clusters (e.g., cluster A 352 and cluster B 354). Classification of a beat segment into a cluster may be based on the segment's beat characteristics and/or may be based on a prediction of the arrhythmia type that produced the biometric data 310 associated with the beat segment. Hence, the output 350 of the evaluation engine 340 may be segments that are classified into clusters based on the segments' beat characteristics - for example, a beat's origin (location of focal arrhythmogenic activity in the heart), the beat's propagation velocity, and the beat's shape descriptors. Alternatively, or in addition, the output 350 of the evaluation engine 340 may be segments that are classified into clusters based on the predicted type of arrhythmia that the segments represent - for example, a specific arrhythmia type, a normal sinus rhythm (NSR), or a mixed arrhythmia. The mapper 360 may then generate maps (e.g., voltage maps or LAT maps). Each map visualizes data that are associated with segments from one cluster (e.g., map 372 visualizes data associated with segments in cluster B 354).

The biometric data 310, provided to the system 300, may contain data measured by the system 100, 200 from a patient during an EP procedure. The biometric data may include BS ECG data, IC ECG data, ablation data, and catheter electrode position data. In an aspect, the BS ECG data may include signals collected from electrodes positioned on a surface of a patient's body; the IC ECG data may include signals collected from electrodes within the patient's body; and the ablation data may include data collected from tissue that has been ablated. Thus, a beat segment 330 may be extracted according to a time window from a BS ECG signal or an IC ECG signal that contains at least one cardiac beat. It should be understood that the signal can be collected from the patient's body using any one of a number of different instruments used to measure electrical activity in the patient's body. As mentioned above, classification of the beat segments 330 into clusters may be based on an arrhythmia type, such as a specific arrhythmia type, an NSR, a mixed arrhythmia, or a combination thereof. For example, specific arrhythmia types may include atrial fibrillation, flutter, or atrial tachycardia. An NSR may be a rhythm of a healthy heart, that is, a properly transmitted electrical pulse from a sinus node. A mixed arrhythmia may include two or more types of arrhythmia as well as one or more types of arrhythmia and an NSR.

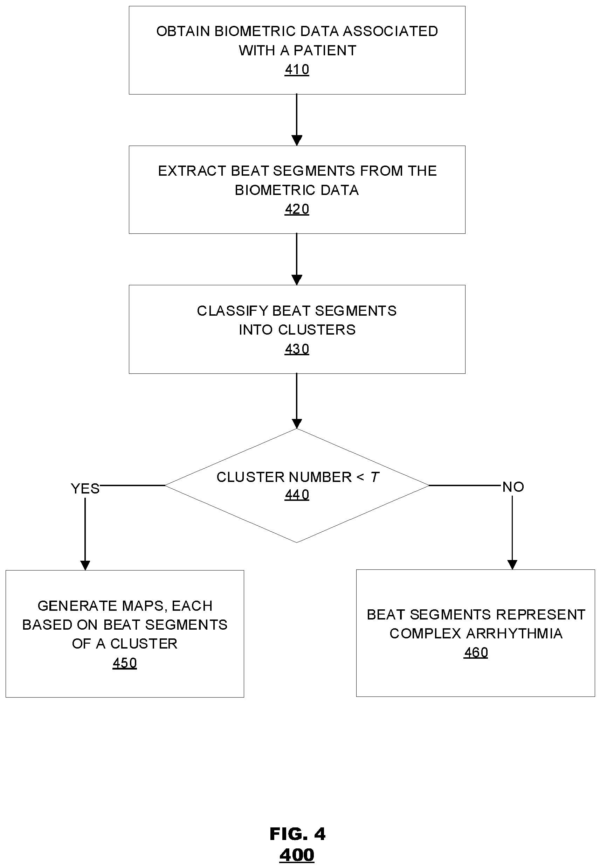

FIG. 4 is a flow chart of an example method 400 for cardiac mapping of data according to clusters, based on which one or more features of the disclosure may be implemented. In step 410, biometric data measured by the system 100, 200 during a medical procedure of a patient are obtained. The biometric data may contain any data related to the heart condition, including, for example, BS ECG data, IC ECG data, ablation data, and catheter electrode position data. The method 400 may extract beat segments from the biometric data in step 420. In an aspect, the segments may be overlapping in time or may be extracted within a rolling time window across the temporal dimension of BS ECG data and/or IC ECG data. In step 430, the segments are classified into clusters. As stated above, classification of a beat segment into a cluster may be based on the segment's beat characteristics and/or may be based on a prediction of arrhythmia type based on the biometric data 310 associated with the beat segment. Next, if the segments are clustered into a number of clusters that is below a threshold number $T$ 440, cardiac maps may be generated in step 450. Each generated map may visualize data associated with segments from one cluster. However, if the segments are clustered into a number of clusters that is equal or above the threshold number $T$ 440, it may be concluded, in step 460, that the beat segments represent a complex arrythmia, a conclusion that may affect the course of the treatment.

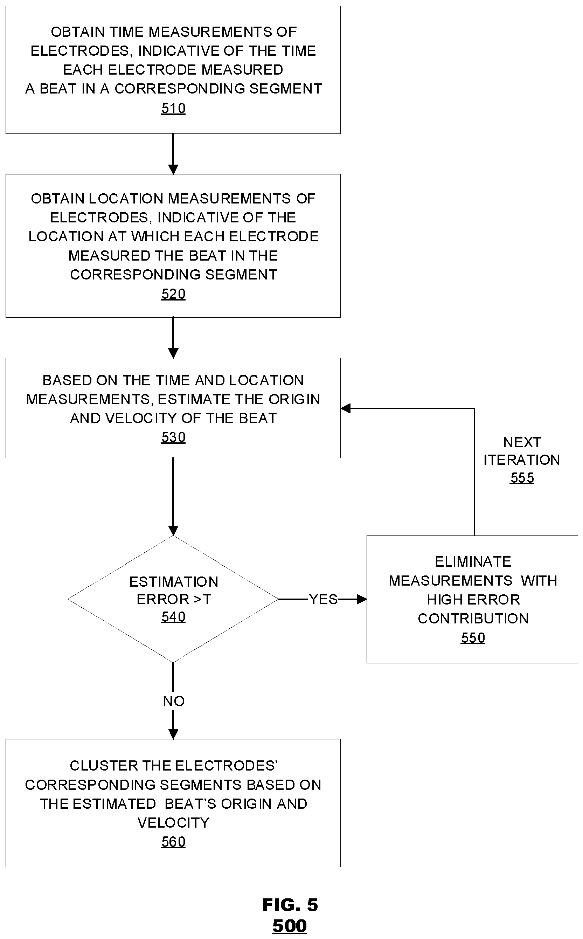

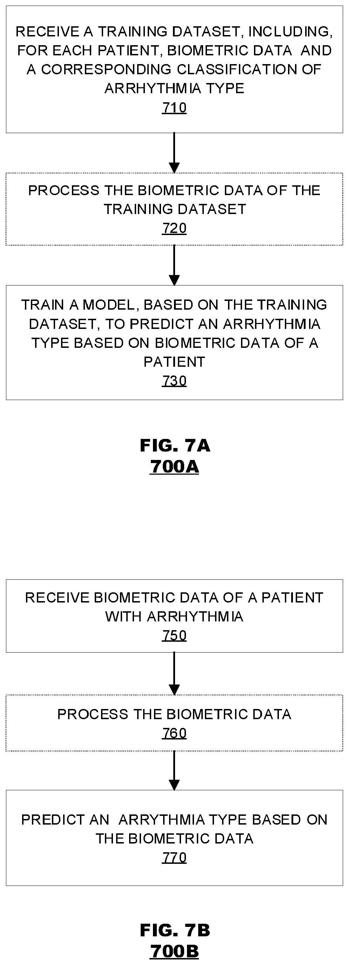

The classification of beat segments into clusters (in step 430 of method 400 or by the evaluation engine 340 of system 300) may be achieved based on clustering algorithms or based on machine learning algorithms. Classifying beat segments into clusters based on beat characteristics, utilizing clustering algorithms, is described herein with reference to FIGS. 5-6. Classifying beat segments into clusters based on the segments associated biometric data, utilizing machine learning algorithms, is described herein with reference to FIGS. 7-8.

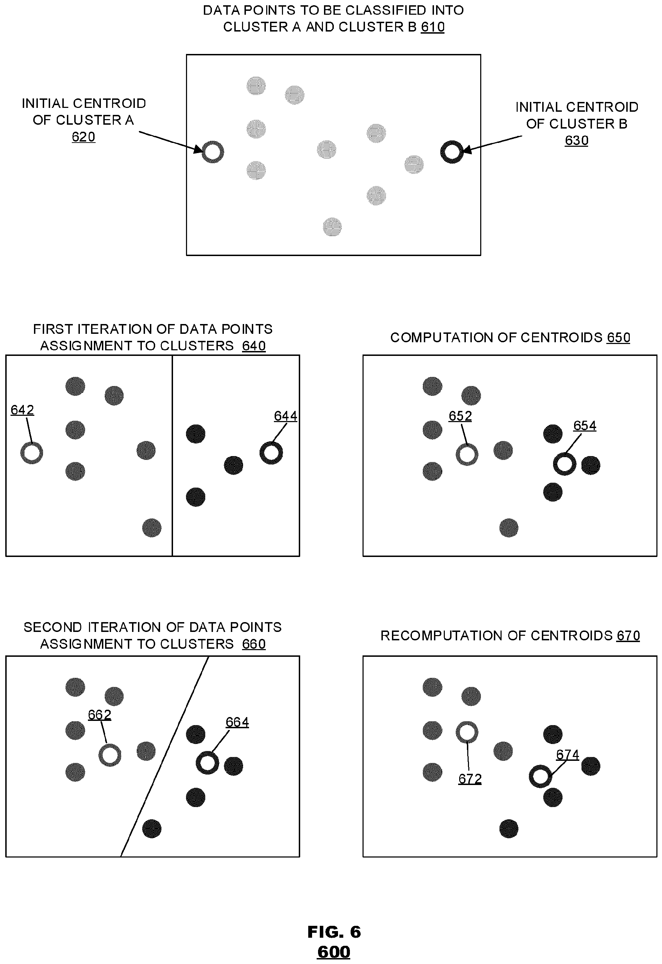

FIG. 5 is a flow chart of an example method 500 for classifying beat segments into clusters based on beat characteristics, based on which one or more features of the disclosure may be implemented. The method 500 may estimate the characteristics (origin and velocity) of a beat represented in a subset of beat segments 335 that were captured by electrodes of a multi-electrode catheter. The method 500 begins in step 510, where time measurements of the electrodes are obtained. A time measurement of an electrode indicates the time the electrode measured a beat in a segment (e.g., 335.m). Then, in step 520, location measurements of the electrodes are obtained. A location measurement of an electrode indicates the location in the heart the electrode was at when it measured the beat in the segment (e.g., 335.m). Based on these time and location measurements, the method 500 estimates in step 530, the origin (location in the heart) of the beat and the beat propagation velocity, as described below in detail. If the estimation error is above a threshold $T$ 540, measurements with high error contribution may be eliminated, in step 550, and the estimation in step 530 may be repeated using only the remaining measurements 555. If the estimation error is equal or below the threshold $T$ 540, the beat's origin estimate and velocity estimate may be used to classify into clusters the subset of beat segments captured by the multi-electrode catheter, as described in reference to FIG. 6.

A technique for estimating the origin and the velocity of a beat (step 530) is described next. The technique estimates a location of focal arrhythmogenic activity in the heart (i.e., origin) and its conduction velocity (i.e., velocity). Electrodes of a multielectrode catheter (e.g., Pentaray® catheter, Constellation catheter, or Coronary Sinus Catheter) may be used by the system 100, 200 to acquire multiple signals from cardiac regions the electrodes are positioned at. In an aspect, a Coronary Sinus catheter may be used to acquired reference measurements. Such reference measurements can be used to link (or calibrate) measurements acquired, for example, by a Pentaray catheter. That is, measurements that were taken by a Pentaray catheter (at different times and locations in the atria) can be measured relative to corresponding measurements taken by a Coronary Sinus catheter, so that the measurements acquired by the Pentaray catheter can be linked to create, for example, a LAT map.

Segments, including an atrial activation (a beat), are extracted from the signals acquired by the electrodes of a multielectrode (i.e., beat segments 330, 420). Associated with each beat segment, or each electrode $i$, is the time $t_i$, the time in which the atrial activation was measured by electrode $i$, and the location of that electrode $(x_i, y_i, z_i)$ at time $t_i$. The activation time of the beat is denoted by $t_0$ and the beat's origin is denoted by $(x_0, y_0, z_0)$. The locations of the electrodes $(x_i, y_i, z_i)$ and the origin of the beat $(x_0, y_0, z_0)$ are typically defined relative to a cardiac reference location, for example at the region of interest within the heart. Thus, the beat may start at a location $(x_0, y_0, z_0)$ in the heart at a time $t_0$, may propagate at a velocity $v$ (i.e., the conduction velocity), and may be measured by electrode $i$ at a location $(x_i, y_i, z_i)$, at time $t_i$.

Hence, with respect to electrode $i$, the beat may travel along a distance $d_i$, defined by

$$d_i = \sqrt{(x_i - x_0)^2 + (y_i - y_0)^2 + (z_i - z_0)^2}$$ and the beat's traveling time may be defined as

$$\frac{d_i}{v} = t_i - t_0 + \varepsilon_i$$, where $\varepsilon_i$ represents the measurement error associated with electrode $i$. An estimate for the beat's origin, $(x_0, y_0, z_0)$, and the beat's velocity $v$ may then be computed, based on measurements from $m$ electrodes, by minimizing the mean squared error: $1/m \sum_{i=1}^{m} (\varepsilon_i)^2$ as follows:

$$J(\theta) = 1/m \sum_{i=1}^{m} (\varepsilon_i)^2 = \frac{1}{m} \sum_{i=1}^{m} (d_i/v - (t_i - t_0))^2, \qquad (1)$$

where $\theta = (x_0, y_0, z_0, t_0, v)$ is the parameter vector to be estimated out of the distance measurements $\{d_i, i = 1 \ldots m\}$ (or equivalently location measurements $\{(x_i, y_i, z_i), i = 1 \ldots m\}$ ) and out of the time measurements $\{t_i, i = 1 \ldots m\}$ of the $m$ electrodes. In an aspect, a regularization term $\varphi$ may be used:

$$J(\theta) = \frac{1}{m} \sum_{i=1}^{m} (d_i/v - (t_i - t_0))^2 + \varphi. \qquad (2)$$

For example, setting the regularization term $\varphi$ to $\frac{\lambda}{2m} (x_0^2 + y_0^2 + z_0^2 + v^2)$ may result in a solution for $\theta = (x_0, y_0, z_0, t_0, v)$ that is closer to the origin of the catheter and that is likely to be within a region of interest within the anatomy of the heart. Minimizing the cost function $J(\theta)$ (to reach at the beat origin $(x_0, y_0, z_0)$ estimate and the beat's propagation velocity estimate) may be accomplished by an optimization algorithm, such as a gradient-descent based optimization.

Based on these estimates, the mean squared error: $1/m \sum_{i=1}^{m} (\varepsilon_i)^2$ may be computed. If the mean squared error is above a threshold $T$ (step 540), electrodes' measurements that are associated with an error $\varepsilon_i$ that is above a predetermined error level may be eliminated (step 550) and the estimation (step 530) may be repeated including only the measurements of the remaining electrodes 555.

FIG. 6 is a diagram of an example clustering method 600, based on which one or more features of the disclosure may be implemented. The method 600 may be used to classify beat segments into clusters based on the segments' beat characteristics - that is, based on the segments' beat's origin in the heart and the beat's velocity (e.g., as estimated by method 500) and/or based on other descriptors, such as shape descriptors. The clusters may be represented by respective centroids in a space defined by the data based on which the classification is performed. For example, the four-dimensional space defined by a beat's origin $(x_0, y_0, z_0, )$ and a beat's velocity $v$.

In FIG. 6, classification of the data points in a two-dimensional space according to a K-means clustering algorithm is demonstrated. As shown, the data points are to be classified into two clusters (K=2) - cluster A and cluster B 610. In a K-means algorithm, the number of clusters should be pre-determined. In an aspect, the number of clusters may be learned by a machine learning algorithm based on a training data (e.g., of beat segments) for which the number of clusters can be validated by a physician. To initialize the K-means algorithm, two initial centroids are determined for each cluster, set apart from each other. The centroids are denoted by hollow circles 620, 630. Then, in a first iteration, the data points may be classified into two clusters based on their similarity to each centroid 640. Thus, data points to the left of the boundary line may be assigned to cluster A 642 and data points to the right of the boundary line may be classified to cluster B 644. Next, the centroids of the clusters are computed 650 based on the data points within each cluster 652, 654. In a second iteration, the data points may again be reclassified into two clusters based on their similarity to each of the centroids 660. Thus, data points to the left of the boundary line may be assigned to cluster A 662 and data points to the right of the boundary line may be assigned to cluster B 664. Next, the centroids of the clusters are recomputed 670 based on the data points within each cluster 672, 674. The process of assigning data points into clusters 640, 660 and recomputing the clusters' centroids 650, 670 may repeat until a maximum iteration number is reached or until there is no change in data points assignments into clusters.

FIG. 7 is a flow chart of an example method for classifying beat segments into clusters based on a prediction of an arrhythmia type, including a training method 700A of a predictor (FIG. 7A) and application method 700B of the trained predictor (FIG. 7B), based on which one or more features of the disclosure may be implemented. The method 700A-B may be employed by the evaluation engine 340 whose execution may be carried out by any processor of the system, 100 or 200, described herein with respect to FIG. 1 or FIG. 2, respectively. In step 710 of the method 700A, a training dataset may be received, including training pairs. Each pair comprises biometric data associated with a patient with an arrhythmia and corresponding classification of the arrhythmia type in that patient. The biometric data of a pair may contain beat segments representative of an arrhythmia type. In step 720, the biometric data of the training dataset may be processed to improve their predictive value and/or to reduce their size. In an aspect, step 720 is not applied to the biometric data. Based on the training dataset provided by step 720 (or step 710), an arrhythmia prediction model may learn the correlation between training pairs of biometric data and their corresponding classifications - that is, in step 730, the model is trained to predict a type of arrhythmia when presented with biometric data of a new patient.

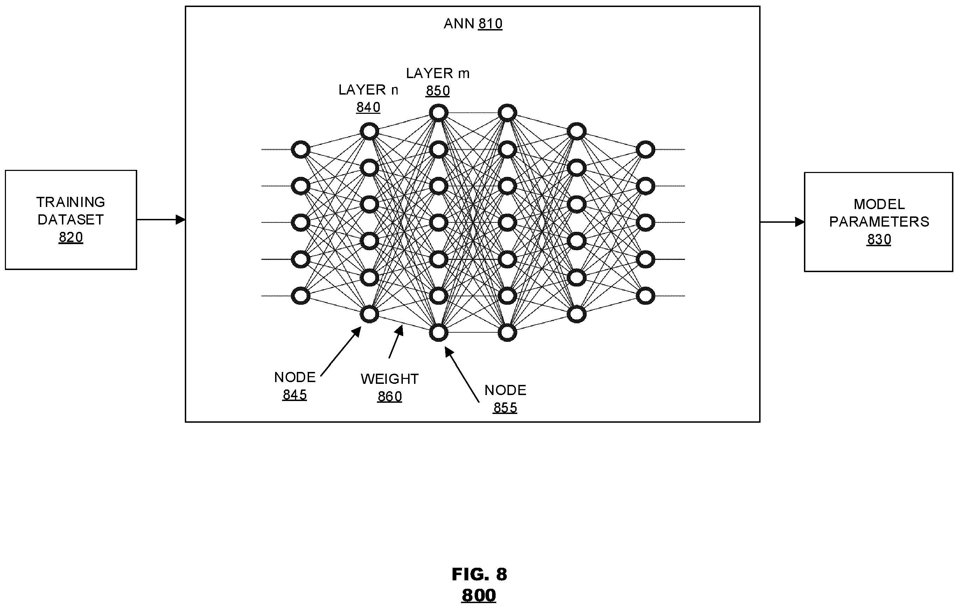

The received training dataset 710 may include biometric data measured by the system, 100 or 200, as described herein with respect to FIG. 1 or FIG. 2. For example, the training dataset 710 may include biometric data derived from measurements obtained during surgical procedures and corresponding classifications that are associated with the outcome of those surgical procedures. For instance, a patient's biometric data may contain BS ECG data, IC ECG data, ablation data, and catheter electrode position data, and may be manually annotated (classified) by a physician as corresponding to a specific arrhythmia type, an NSR, a mixed arrhythmia, or a combination thereof. As explained in reference to FIG. 8, the outcome of training a model (step 730) is model parameters (weights) that are used when applying the model for prediction. In an aspect, the arrhythmia prediction model may be updated (the weights may be recalculated) in real time as more training data becomes available (and added to the training dataset 710), e.g., following a surgical procedure.

Hence, in step 750 of the method 700B, the trained arrhythmia prediction model may be applied to biometric data 750 measured from a patient that is currently undergoing a medical procedure, for example, biometric data that was measured during a surgical procedure. The biometric data 750 may include BS ECG data, IC ECG data, and catheter electrode position data. In step 760, the patient's biometric data may be processed in the same manner the training biometric data were processed in step 720 of FIG. 7A. In an aspect, steps 720 and 760 are not applied. Then, in step 770, the trained arrhythmia prediction model may be applied to predict the patient's arrhythmia type, that is, a specific arrhythmia type, an NSR, a mixed arrhythmia, or a combination thereof. The predicted arrythmia type may be used to classify the corresponding beat segment into a cluster in accordance to step 430 of FIG. 4.

FIG. 8 is a functional block diagram of an example machine learning system 800, based on which one or more features of the disclosure may be implemented. Various machine learning systems 800 may be used to train and to apply the arrhythmia predictor employed by the evaluation engine 340 of FIG. 3. For example, the machine learning system 800 may be based ANN of various architectures, such as a convolutional neural network (CNN) or a recurrent neural network (RNN), an example of which is the LSTM network. Generally, neural networks are trained to predict information of interest based on observations. A neural network is trained via a supervised learning process, through which correlations between example pairs (i.e., observations and corresponding information of interest) are learned.

In an aspect, the ANN 810 may be a CNN. A CNN is useful in learning patterns from data provided in a spatiotemporal format, such as ECG data. Generally, a CNN may employ convolution operations, using kernels, across several layers. Each layer, e.g., layer $n$ 840, in the network may process data from an image at its input and may generate a processed image to be processed by the next layer, e.g., layer $m$ 850. Convolutional kernels that are applied in earlier layers in the network may integrate information from neighboring data elements more efficiently than convolutional kernels that are applied in later layers in the network. Therefore, correlations among data elements that are closely positioned in the image may be better learned in a CNN.

Typically, a neural network 810 comprises nodes ("neurons") that are connected according to a given architecture. For example, in a given architecture, the nodes may be arranged in layers - that is, the output of nodes in one layer, e.g., layer $n$ 840, feed the input of nodes in the next layer connected to it, e.g., layer $m$ 850. A node $j$ 855 of a layer $m$ 850 (i.e., $m_j$) is typically connected to a node $i$ 845 of a layer $n$ 540 (i.e., $n_i$) with a certain strength or a certain weight: $w(m_j, n_i)$ 860. Hence, the weights $\{w(m_j, n_i)\}$ associated with a network's inter-node connections ("synaptic weights") parametrize the neural network model. Training the neural network, then, can be viewed as specializing the network by determining the weights (parameters) of the network, that is, determining the model parameters 830.

The manner in which a neural network 810 processes data may be described as follows. Input data may be fed to nodes in the first layer of a neural network so that each node in the first layer receives a weighted combination of the input data (or a weighted combination of a subset of the input data). Then, each node's inputted weighted combination is translated according to an activation function of the node, resulting in the node's output data. Next, output data from each node in the first layer may be fed to nodes in the second layer of the neural network so that each node in the second layer receives a weighted combination of the outputs of nodes in the first layer (or a weighted combination of the outputs of a subset of the nodes in the first layer). Then, each node's inputted weighted combination is translated according to an activation function of the node, resulting in the node's output data. The output data from nodes of the second layer are then propagated and similarly processed in the other intermediate layers of the network, where the last layer provides the network's output data. Hence, a neural network is typically characterized by the structure of its nodes and these nodes' activation functions. The weights associated with the inter-node connections (the network parameters or the model parameters 830) are learned by an iterative training process, e.g., a backpropagation algorithm, according to training parameters (e.g., a learning rate and a cost function) and based on a training dataset 820.

A training dataset 820, based on which a neural network model, 810, may be trained may include pairs of example data, such as observation data (e.g., measurements collected during surgical procedures) and corresponding information of interest to be predicted by the model (e.g., outcomes of the surgical procedures). For example, the temperature data of the heart (observation data) may be collected and may be correlated (by the training process) with outcomes of a heart procedure (information of interest to be predicted). Once the model parameters are determined by the training process, the model can be applied to predict the information of interest based on a new observation. For example, in the case of the heart, based on an input of temperature during a procedure (e.g., between 97.7 - 100.2 degrees Celsius) the model output may be a prediction of the outcome of the procedure. Such a prediction is based on the correlation between the temperature and the procedure's outcome that was learned by the neural network model based on the training dataset.

In accordance with aspects of the present disclosure, the evaluation engine 340, may train a machine learning model 810 and may apply the trained model to predict the classification of an arrhythmia condition in a patient. Algorithms disclosed herein may be applied to train models based on a training dataset 820 (obtained by various modalities from patients experiencing an arrhythmia condition) and corresponding classification of the patients' arrhythmia condition.

The flowchart and block diagrams in the Figures illustrate the architecture, functionality, and operation of possible implementations of systems and computer program products according to various aspects of the present invention. In this regard, each block in the flowchart or block diagrams may represent a module, segment, or portion of instructions, which includes one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The methods described herein, which alone are not part of the invention defined by the claims, may be implemented in a computer program, software, or firmware incorporated in a computer-readable medium for execution by a computer or processor as claimed hereinafter. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random-access memory (RAM), a read-only memory (ROM), an erasable programmable read-only memory (EPROM or Flash memory), a static random-access memory (SRAM), and a memory stick. A processor in association with software may be used to implement a radio frequency transceiver for use in a WTRU, UE, terminal, base station, RNC, or any host computer.

**Claims**

1. A system (100) for cardiac mapping, comprising:

    at least one processor (161); and
    memory (162) storing instructions that, when executed by the at least one processor, cause the system to:

        receive biometric data obtained from a patient (125),
        extract beat segments from the biometric data,
        classify the beat segments into clusters, and
        generate at least one map based on data associated with beat segments in one of the clusters, **characterized in that** each cluster represents an arrhythmia type and the instructions cause the system to generate said map in response to the beat segments being classified into a number of clusters that is below a threshold number.

2. A non-transitory computer readable medium comprising instructions executable by at least one processor to cause the at least one processor to:

        receive biometric data obtained from a patient (125),
        extract beat segments from the biometric data,
        classify the beat segments into clusters, and
        generate at least one map based on data associated with beat segments in one of the clusters, **characterized in that** each cluster represents an arrhythmia type and the instructions cause the at least one processor to generate said map in response to the beat segments being classified into a number of clusters that is below a threshold number.

3. The system (100) of claim 1 or the non-transitory computer readable medium of claim 2, wherein the biometric data comprise at least one of body surface electrocardiogram data, intracardiac electrocardiogram data, or ablation data.

4. The system (100) of claim 1 or the non-transitory computer readable medium of claim 2, wherein the biometric data comprise catheter electrode position data.

5. The system (100) of claim 1 or the non-transitory computer readable medium of claim 2, wherein the biometric data comprise measurements acquired by a catheter (105) and corresponding reference measurements acquired by a Coronary Sinus catheter, and wherein the reference measurements are used to link the measurements acquired by the catheter.

6. The system (100) of claim 1 or the non-transitory computer readable medium of claim 2, wherein the arrhythmia type comprises a specific arrhythmia type, a normal sinus rhythm, a mixed arrhythmia, or a combination thereof.

7. The system (100) of claim 1 or the non-transitory computer readable medium of claim 2, wherein the classifying comprises:

        for each of the beat segments, estimating one or more beat characteristics; and
        classifying the beat segments into the clusters based on their respective beat characteristics.

8. The system (100) or non-transitory computer readable medium of claim 6, wherein the beat characteristics comprise one or more of a shape-descriptor, an origin, or a propagation velocity.

9. The system (100) or non-transitory computer readable medium of claim 6, wherein:
   a subset of the beat segments represents a beat **characterized by** an origin in the heart and a propagation velocity, each beat segment in the subset being captured by a corresponding electrode of a multielectrode catheter.

10. The system (100) or non-transitory computer readable medium of claim 8, wherein:
    the estimating comprises estimating the origin and the propagation velocity of the beat based on time measurements, each indicative of a time an electrode of the multielectrode catheter measures the beat in a corresponding beat segment of the subset, and based on location measurements, each indicative of a location of the electrode at the time the beat was measured by the electrode.

**11.** The system (100) of claim 1 or the non-transitory computer readable medium of claim 2, wherein the classifying comprises:

> for each of the beat segments, predicting, based on biometric data associated with the beat segment, a respective arrhythmia type using a machine learning model (810); and
> classifying the beat segments into the clusters based on their respective predicted arrhythmia type.

**12.** The system (100) or non-transitory computer readable medium of claim 10, further comprising instructions that cause the system or the at least one processor to:

> receive a training dataset (820) associated with past patients, wherein the training dataset for each past patient comprises:
>
> > beat segments extracted from biometric data obtained from the past patient, and
> > a classification of arrhythmia type for each beat segment; and
>
> train the machine learning model, based on the training dataset, to predict an arrhythmia type associated with a beat segment obtained from the patient.

**Patentansprüche**

**1.** System (100) zur kardialen Kartierung, umfassend:

> wenigstens einen Prozessor (161); und
> Speicher (162), welcher Anweisungen speichert, die, wenn sie von dem wenigstens einen Prozessor ausgeführt werden, das System hierzu veranlassen:
>
> > Empfangen biometrischer Daten, die von einem Patienten (125) erhalten werden,
> > Extrahieren von Schlagsegmenten aus den biometrischen Daten, Klassifizieren der Schlagsegmente in Cluster und
> > Generieren von wenigstens einer Karte basierend auf Daten, die mit Schlagsegmenten in einem der Cluster verknüpft sind, **dadurch gekennzeichnet, dass** jeder Cluster einen Arrhythmietyp darstellt und die Anweisungen das System veranlassen, in Reaktion darauf, dass die Schlagsegmente in eine Anzahl von Clustern klassifiziert werden, die unter einer Schwellwertanzahl liegt, die Karte zu generieren.

**2.** Nicht-transitorisches computerlesbares Medium, umfassend Anweisungen, die von wenigstens einem Prozessor ausführbar sind, um den wenigstens einen Prozessor hierzu zu veranlassen:

> Empfangen biometrischer Daten, die von einem Patienten (125) erhalten werden,
> Extrahieren von Schlagsegmenten aus den biometrischen Daten, Klassifizieren der Schlagsegmente in Cluster und
> Generieren von wenigstens einer Karte basierend auf Daten, die mit Schlagsegmenten in einem der Cluster verknüpft sind, **dadurch gekennzeichnet, dass** jeder Cluster einen Arrhythmietyp darstellt und die Anweisungen den wenigstens einen Prozessor veranlassen, in Reaktion darauf, dass die Schlagsegmente in eine Anzahl von Clustern klassifiziert werden, die unter einer Schwellwertanzahl liegt, die Karte zu generieren.

**3.** System (100) nach Anspruch 1 oder nicht-transitorisches computerlesbares Medium nach Anspruch 2, wobei die biometrischen Daten wenigstens eines von Körperoberflächen-Elektrokardiogrammdaten, intrakardialen Elektrokardiogrammdaten oder Ablationsdaten umfasst.

**4.** System (100) nach Anspruch 1 oder nicht-transitorisches computerlesbares Medium nach Anspruch 2, wobei die biometrischen Daten Katheterelektroden-Positionsdaten umfassen.

**5.** System (100) nach Anspruch 1 oder nicht-transitorisches computerlesbares Medium nach Anspruch 2, wobei die biometrischen Daten Messungen, die durch einen Katheter (105) bezogen werden, und entsprechende Referenzmessungen, die durch einen Koronarsinus (Coronary Sinus)-Katheter bezogen werden, umfassen und wobei die Referenzmessungen verwendet werden, um die durch den Katheter bezogenen Messungen zu verbinden.

**6.** System (100) nach Anspruch 1 oder nicht-transitorisches computerlesbares Medium nach Anspruch 2, wobei der Arrhythmietyp einen spezifischen Arrhythmietyp, einen normalen Sinusrhythmus, eine gemischte Arrhythmie oder eine Kombination davon umfasst.

**7.** System (100) nach Anspruch 1 oder nicht-transitorisches computerlesbares Medium nach Anspruch 2, wobei das Klassifizieren umfasst:

für jedes der Schlagsegmente, Schätzen eines oder mehrerer Schlagcharakteristika; und
Klassifizieren der Schlagsegmente in die Cluster basierend auf deren jeweiligen Schlagcharakteristika.

**8.** System (100) oder nicht-transitorisches computerlesbares Medium nach Anspruch 6, wobei die Schlagcharakteristika eines oder mehrere von einem Formdesktriptor, einem Ursprung oder einer Ausbreitungsgeschwindigkeit umfassen.

**9.** System (100) oder nicht-transitorisches computerlesbares Medium nach Anspruch 6, wobei:
eine Teilmenge der Schlagsegmente einen Schlag darstellt, **gekennzeichnet durch** einen Ursprung im Herzen und einer Ausbreitungsgeschwindigkeit, wobei jedes Schlagsegment in der Teilmenge von einer entsprechenden Elektrode eines Mehrelektrodenkatheters erfasst wird.

**10.** System (100) oder nicht-transitorisches computerlesbares Medium nach Anspruch 8, wobei:
das Schätzen ein Schätzen des Ursprungs und der Ausbreitungsgeschwindigkeit des Schlags basierend auf Zeitmessungen umfasst, von denen jede eine Zeit angibt, für die eine Elektrode des Mehrelektrodenkatheters den Schlag in einem entsprechenden Schlagsegment der Teilmenge misst, und basierend auf Positionsmessungen, von denen jede eine Position der Elektrode zu dem Zeitpunkt angibt, an dem der Schlag von der Elektrode gemessen wurde.

**11.** System (100) nach Anspruch 1 oder nicht-transitorisches computerlesbares Medium nach Anspruch 2, wobei das Klassifizieren umfasst:

für jedes der Schlagsegmente, Vorhersagen, basierend auf biometrischen Daten, die mit dem Schlagsegment verknüpft sind, eines jeweiligen Arrhythmietyps unter Verwendung eines Maschinenlernmodells (810); und
Klassifizieren der Schlagsegmente in die Cluster basierend auf dem jeweiligem vorhergesagten Arrhythmietyp.

**12.** System (100) oder nicht-transitorisches computerlesbare Medium nach Anspruch 10, ferner umfassend Anweisungen, die das System oder den wenigstens einen Prozessor hierzu veranlassen:
Empfangen eines Trainingsdatensatzes (820), der mit früheren Patienten verknüpft ist, wobei der Trainingsdatensatz für jeden früheren Patienten umfasst:

Schlagsegmente, die aus biometrischen Daten extrahiert werden, welche von dem früheren Patienten erhalten werden, und
eine Klassifizierung eines Arrhythmietyps für jedes Schlagsegment; und
Trainieren des Maschinenlernmodells, basierend auf dem Trainingsdatensatz, um einen Arrhythmietyp vorherzusagen, der mit einem Schlagsegment verknüpft ist, das von dem Patienten erhalten wird.

## Revendications

**1.** Système (100) de cartographie cardiaque comprenant :

au moins un processeur (161) ; et
une mémoire (162) stockant des instructions qui, lorsqu'elles sont exécutées par l'au moins un processeur, amènent le système à :

recevoir des données biométriques obtenues d'un patient (125),
extraire des segments de battement des données biométriques,
classer les segments de battement en groupes, et
générer au moins une carte basée sur les données associées aux segments de battements dans l'un des groupes, **caractérisée en ce que** chaque groupe représente un type d'arythmie et que les instructions amènent le système à générer ladite carte en réponse à la classification des segments de battements dans un nombre de groupes inférieur à un nombre seuil.

2. Support lisible par ordinateur non transitoire comprenant des instructions exécutables par au moins un processeur afin d'amener l'au moins un processeur à :

recevoir des données biométriques obtenues d'un patient (125),
extraire des segments de battement des données biométriques,
classer les segments de battement en groupes, et
générer au moins une carte basée sur les données associées aux segments de battements dans l'un des groupes, **caractérisée en ce que** chaque groupe représente un type d'arythmie et que les instructions amènent l'au moins un processeur à générer ladite carte en réponse à la classification des segments de battements dans un nombre de groupes inférieur à un nombre seuil.

3. Système (100) selon la revendication 1 ou support lisible par ordinateur non transitoire selon la revendication 2, les données biométriques comprenant au moins certaines données parmi des données d'électrocardiogramme à la surface du corps, des données d'électrocardiogramme intracardiaque ou des données d'ablation.

4. Système (100) selon la revendication 1 ou support lisible par ordinateur non transitoire selon la revendication 2, les données biométriques comprenant des données sur la position des électrodes du cathéter.

5. Système (100) selon la revendication 1 ou support lisible par ordinateur non transitoire selon la revendication 2, les données biométriques comprenant des mesures acquises par un cathéter (105) et des mesures de référence correspondantes acquises par un cathéter du sinus coronaire, et les mesures de référence étant utilisées pour relier les mesures acquises par le cathéter.

6. Système (100) selon la revendication 1 ou support lisible par ordinateur non transitoire selon la revendication 2, le type d'arythmie comprenant un type d'arythmie spécifique, un rythme sinusal normal, une arythmie mixte ou une combinaison de ceux-ci.

7. Système (100) selon la revendication 1 ou support lisible par ordinateur non transitoire selon la revendication 2, la classification comprenant :

pour chacun des segments de battement, l'estimation d'une ou plusieurs caractéristiques de battement ; et
la classification des segments de battement dans les groupes en fonction de leurs caractéristiques de battement respectives.

8. Système (100) ou support lisible par ordinateur non transitoire selon la revendication 6, les caractéristiques de battement comprenant un ou plusieurs descripteurs de forme, une origine ou une vitesse de propagation.

9. Système (100) ou support lisible par ordinateur non transitoire selon la revendication 6, un sous-ensemble de segments de battements représentant un battement **caractérisé par** une origine dans le cœur et une vitesse de propagation, chaque segment de battement du sous-ensemble étant capturé par une électrode correspondante d'un cathéter multiélectrode.

10. Système (100) ou support lisible par ordinateur non transitoire selon la revendication 8, l'estimation comprenant l'estimation de l'origine et de la vitesse de propagation du battement sur la base de mesures temporelles, chacune indiquant le moment où une électrode du cathéter multiélectrode mesure le battement dans un segment de battement correspondant du sous-ensemble, et sur la base de mesures d'emplacement, chacune indiquant l'emplacement de l'électrode au moment où le battement a été mesuré par l'électrode.

11. Système (100) selon la revendication 1 ou support lisible par ordinateur non transitoire selon la revendication 2, la classification comprenant :

pour chacun des segments de battement, la prédiction, sur la base des données biométriques associées au segment de battement, d'un type d'arythmie respectif à l'aide d'un modèle d'apprentissage automatique (810) ; et
la classification des segments de battement dans les groupes en fonction de leur type d'arythmie prédit respectif.

**12.** Système (100) ou support lisible par ordinateur non transitoire selon la revendication 10, comprenant en outre des instructions qui amènent le système ou l'au moins un processeur à :

recevoir un ensemble de données d'entraînement (820) associé à des patients passés, l'ensemble de données d'entraînement pour chaque patient passé comprenant :

des segments de battements extraits des données biométriques obtenues auprès du patient passé, et une classification du type d'arythmie pour chaque segment de battement ; et

entraîner le modèle d'apprentissage automatique, sur la base de l'ensemble de données d'entraînement, pour prédire un type d'arythmie associé à un segment de battement obtenu auprès du patient.

FIG. 1
100

# MONITORING AND PROCESSING SYSTEM 205

| SENSOR<br>210 | PROCESSOR<br>220 | MEMORY<br>230 |

| INPUT DEVICE<br>240 | OUTPUT DEVICE<br>250 | TRANSCEIVER<br>260 |

NETWORK
270

LOCAL
SYSTEM
280

NETWORK
285

REMOTE
SYSTEM
290

**FIG. 2**
**200**

EP 3 960 085 B1

FIG. 3
300

21

OBTAIN BIOMETRIC DATA ASSOCIATED
WITH A PATIENT
410

EXTRACT BEAT SEGMENTS FROM THE
BIOMETRIC DATA
420

CLASSIFY BEAT SEGMENTS
INTO CLUSTERS
430

YES — CLUSTER NUMBER < $T$
440 — NO

GENERATE MAPS, EACH
BASED ON BEAT SEGMENTS
OF A CLUSTER
450

BEAT SEGMENTS REPRESENT
COMPLEX ARRHYTHMIA
460

**FIG. 4**
**400**

OBTAIN TIME MEASUREMENTS OF
ELECTRODES, INDICATIVE OF THE TIME
EACH ELECTRODE MEASURED
A BEAT IN A CORRESPONDING SEGMENT
510

OBTAIN LOCATION MEASUREMENTS OF
ELECTRODES, INDICATIVE OF THE
LOCATION AT WHICH EACH ELECTRODE
MEASURED THE BEAT IN THE
CORRESPONDING SEGMENT
520

BASED ON THE TIME AND LOCATION
MEASUREMENTS, ESTIMATE THE ORIGIN
AND VELOCITY OF THE BEAT
530

NEXT
ITERATION
555

ESTIMATION
ERROR >T
540

YES

ELIMINATE
MEASUREMENTS WITH
HIGH ERROR
CONTRIBUTION
550

NO

CLUSTER THE ELECTRODES'
CORRESPONDING SEGMENTS BASED ON
THE ESTIMATED BEAT'S ORIGIN AND
VELOCITY
560

**FIG. 5**
**500**

DATA POINTS TO BE CLASSIFIED INTO
CLUSTER A AND CLUSTER B 610

INITIAL CENTROID
OF CLUSTER A
620

INITIAL CENTROID
OF CLUSTER B
630

FIRST ITERATION OF DATA POINTS
ASSIGNMENT TO CLUSTERS 640

642

644

COMPUTATION OF CENTROIDS 650

652

654

SECOND ITERATION OF DATA POINTS
ASSIGNMENT TO CLUSTERS 660

662

664

RECOMPUTATION OF CENTROIDS 670

672

674

**FIG. 6**
**600**

RECEIVE A TRAINING DATASET, INCLUDING, FOR EACH PATIENT, BIOMETRIC DATA AND A CORRESPONDING CLASSIFICATION OF ARRHYTHMIA TYPE
710

PROCESS THE BIOMETRIC DATA OF THE TRAINING DATASET
720

TRAIN A MODEL, BASED ON THE TRAINING DATASET, TO PREDICT AN ARRHYTHMIA TYPE BASED ON BIOMETRIC DATA OF A PATIENT
730

**FIG. 7A**
**700A**

RECEIVE BIOMETRIC DATA OF A PATIENT WITH ARRHYTHMIA
750

PROCESS THE BIOMETRIC DATA
760

PREDICT AN ARRYTHMIA TYPE BASED ON THE BIOMETRIC DATA
770

**FIG. 7B**
**700B**

ANN <u>810</u>

LAYER m <u>850</u>

LAYER n <u>840</u>

TRAINING DATASET <u>820</u>

MODEL PARAMETERS <u>830</u>

NODE <u>845</u>

WEIGHT <u>860</u>

NODE <u>855</u>

**FIG. 8**
**<u>800</u>**

**REFERENCES CITED IN THE DESCRIPTION**

**Patent documents cited in the description**

- US 20150065836 A1
- US 20130096449 A1
- US 20190223808 A1